# EUROPÄISCHE PATENTSCHRIFT

(11) **EP 0 424 762 B1**
(45) Veröffentlichungstag und Bekanntmachung des Hinweises auf die Patenterteilung: **08.12.1993**
(21) Anmeldenummer: 90119659.2
(22) Anmeldetag: 13.10.1990
(51) Int. Cl.: C07C 303/14

(54) **Verfahren zur Sulfoxidation von n-Paraffinen**
Process for the sulfoxidation of n-paraffins
Procédé de sulfoxydation de n-paraffines

(30) Priorität: 26.10.1989 DE 3935642
(43) Veröffentlichungstag der Anmeldung: 02.05.1991
(73) Patentinhaber: BAYER AG, 51368 Leverkusen (DE)
(72) Erfinder: Käsbauer, Josef, Dr., W-5632 Wermelskirchen 2 (DE); Fiege, Helmut, Dr., W-5090 Leverkusen 1 (DE)

(56) Entgegenhaltungen:
- DE-A- 1 806 883
- DE-A- 1 910 860
- DE-C- 910 165
- FR-A- 1 461 721
- US-A- 3 666 797

## Beschreibung

Die vorliegende Erfindung betrifft ein neues Verfahren zur Sulfoxidation von n-Paraffinen mit Schwefeldioxid und Sauerstoff unter Belichtung mit UV-Strahlung enthaltendem Licht in Gegenwart einer solchen Menge Wasser, daß das Sulfoxidationsgemisch in homogener Phase vorliegt.

Unter den verschiedenen Verfahrensvarianten zur Sulfoxidation von n-Paraffinen zeigt das sogenannte Licht-Wasser-Verfahren aus Wirtschaftlichkeits- und Qualitätsgründen die meisten Vorteile (Chemie in unserer Zeit 13 (1979), 157). Durch Zusatz von Wasser wird die intermediär auftretende Paraffinpersulfonsäure mit Schwefeldioxid zu Paraffinsulfonsäure und Schwefelsäure umgesetzt. Die zugesetzte Wassermenge ist üblicherweise so groß, das während der Sulfoxidation ein Zweiphasensystem vorliegt.

Aus DE-PS 910 165 ist eine weitere Variante der Sulfoxidation unter Wasserzusatz bekannt, wobei Paraffin und Wasser durch einen sehr großen Durchsatz der Reaktionsgase emulgiert werden. Das Wasser liegt jedoch bei dieser Variante nicht ideal verteilt vor. Dies wirkt sich negativ auf die unerwünschte Bildung von Paraffin-Disulfonsaüre aus, die aus Qualitätsgründen auf 10 Gew.-% begrenzt werden muß. Diese Einschränkung erlaubt nur einen sehr kleinen Paraffinumsatz mit Nachteilen bezüglich der Raum-Zeit-Ausbeute. In H. G. Hauthal, Alkansulfonate (1985) wird hierzu ein Umsatz von 1 % genannt. In der genannten DE-OS 910 165 wird ein Umsatz von etwa 0,6 % genannt, in EP 282 962 von etwa 1,5 %.

Aussagen zur zugesetzten Wassermenge werden in DE-PS 910 165 und DE-AS 19 10 860 gemacht. Danach soll die dem Umsetzungsgemisch zugesetzte Wassermenge so bemessen sein, daß die Konzentration der entstehenden Schwefelsaüre in der Wasserphase nicht über 20 % steigt; diese Aussage wird in den genannten Schriften nicht begründet.

Aus der DE-OS 1 806 883 ist ein Verfahren zur Herstellung von Monosulfonsäuren aus paraffinischen Kohlenwasserstoffen mit Schwefeldioxid und Sauerstoff bekannt, bei dem man die Reaktion in homogener flüssiger Phase durchführt, wobei die Konzentration an Schwefeldioxid 7 bis 23 %, die Reaktionstemperatur 17 bis 37°C und der Umsatz des eingesetzten Kohlenwasserstoffs 1 bis 7 % beträgt. Bei diesem Verfahren ist während der Reaktion kein Wasser zugegen; Wasser wird erst zur Aufarbeitung des Reaktionsgemisches zugefügt. Mit diesem Verfahren erhält man bei einem Paraffinumsatz von 8 % eine Sulfonsäuremasse, die 18,4 % Disulfonsäure enthält bzw. bei einem Paraffinumsatz von 8,5 % eine Disulfonsäuremasse, die 13,5 % Disulfonsäure enthält.

Es bestand daher der Wunsch, ein Verfahren zur Sulfoxidation von n-Paraffinen mit Schwefeldioxid und Sauerstoff unter UV-Belichtung und Wasserzusatz bereitzustellen, bei dem die Reaktion unter technisch verbesserten Bedingungen auch bei höherem Umsatz mit großer Selektivität Paraffinsulfonsäure liefert.

Überraschenderweise wurde bei der Sulfoxidation ein sehr enger, umsatzabhängiger Bereich der Wasserkonzentration gefunden, bei dem das Sulfoxidationsgemisch als wasserklare, homogene Reaktionslösung vorliegt. Vorteilhafterweise wird in diesem Bereich Paraffin auch bei wesentlich höherem Umsatz mit großer Selektivität zu Paraffinsulfonsäure umgesetzt.

Es wurde demnach ein Verfahren zur Herstellung von aliphatischen Sulfonsäuren durch Umsetzung von n-Paraffinen mit Schwefeldioxid und Sauerstoff in Gegenwart von Wasser und unter Belichtung mit UV-Strahlung enthaltendem Licht (Sulfoxidation) gefunden, das dadurch gekennzeichnet ist, daß die Sulfoxidation nach Anlaufen der Reaktion und Erreichung des stationären Zustandes in homogener Phase durchgeführt wird.

Es ist selbstverständlich möglich, die Wassermenge empirisch zu bestimmen, die bei fortschreitendem Paraffinumsatz dem Reaktionsgemisch insgesamt zuzusetzen ist, um die erfindungsgemäß erforderliche homogene Phase aufrechtzuerhalten. Es ist jedoch bevorzugt, die jeweilig zugesetzte Wassermenge vom jeweilig erreichten Paraffinumsatz durch die Formel

H₂O[Gew.-%] = Paraffinumsatz [Mol-%]·0,93 + (1,67±0,5),

wobei das jeweilig insgesamt dem Reaktionsgemisch zugesetzte Wasser in Gewichtsprozent, bezogen auf die zu Reaktionsbeginn vorhandene Gewichtsmenge an Paraffin und die Gesamtmasse an zugesetztem Wasser, eingesetzt wird und der Paraffinumsatz in Mol -%, bezogen auf die zu Reaktionsbeginn vorhandene Gewichtsmenge an Paraffin, berechnet wird.

Dieser Formelzusammenhang zur Erhaltung der homogenen Phase gilt nach Anlaufen der Reaktion und Erreichung des stationären Zustandes.

Der Paraffinumsatz wird durch den Sauerstoffverbrauch berechnet, also durch die Differenz zwischen dem in das Reaktionsgemisch hineingegebenen Sauerstoffs und dem im Abgas bestimmten Sauerstoffs. Zur Einhaltung einer auf max. 10 Gew.-% an Paraffin-Disulfonsäure begrenzten Spezifikation wird das erfindungsgemäße Verfahren bevorzugt in einem Umsatzbereich von 0,5 bis 12 Mol-%, bezogen auf die zu Reaktionsbeginn vorhandene Gewichtsmenge an Paraffin, durchgeführt. In bevorzugter Weise wird das erfindungsgemäße Verfahren in einem Umsatzbereich von 2,5 bis 10 Mol -% durchgeführt.

Das erfindungsgemäße Verfahren kann zum Anlaufen der Reaktion mit einem etwas höheren Wassergehalt begonnen werden, als es der obigen Formel entspricht, beispielsweise mit einer Menge von 1,17 bis 5 Gew.-% Wasser, die vorgelegt werden bzw. zu Beginn der Reaktion im Reaktionsgemisch vorliegen. In bevorzugter Weise werden 1,17 bis 4 Gew.-% Wasser vorgelegt bzw. liegen zu Beginn im Reaktionsgemisch vor. Im weiteren Verlauf des erfindungsgemäßen Verfahrens, beispielsweise ab 1 Mol-% Umsatz, bevorzugt ab 1,5 Mol-%, spätestens jedoch ab 3 Mol -%, mündet die Reaktionsführung in einen stationären Zustand mit homogener Phase und wird ab Erreichung dieses stationären Zustandes im Sinne der obigen Formel weitergeführt.

Die Steuerung der Wassermenge zur Erreichung der gemäß obiger Formel erforderlichen insgesamt zugesetzten Wassermenge erfolgt in der obengenannten Weise über die durch die Reaktion verbrauchte Sauerstoffmenge, die der umgesetzten Paraffinmenge äquivalent ist. Das ebenfalls zur Umsetzung benötigte Schwefeldioxid löst sich gut im Reaktionsgemisch. Es liegt in leichtem Überschuß, bezogen auf die verbrauchte Sauerstoffmenge, vor, beispielsweise in einem Überschuß von 1 bis 20 Mol -%. Etwa überschüssiges SO₂ wird erst bei der Aufarbeitung des Reaktionsgemisches aus diesem Gemisch entfernt und kann hierbei in geeigneter Weise aufgefangen und recyclisiert werden. Im Reaktor erreicht man dadurch eine nahezu abgasfreie Fahrweise, bzw. eine Fahrweise ohne entsorgungsbedürftiges Abgas.

Der Sauerstoff für das erfindungsgemäße Verfahren kann reiner Sauerstoff sein. Es ist möglich, das erfindungsgemäße Verfahren so zu betreiben, daß der zugesetzte Sauerstoff nahezu völlig verbraucht wird, so daß nur zur sicheren Bestimmung dieses Sauerstoffverbrauchs ein geringer Durchschlag an Sauerstoff auf der Abgasseite erforderlich ist, um Messungen zur Steuerung des Wasserzusatzes zu ermöglichen. In bevorzugter Weise kann jedoch wegen des ausbleibenden Durchschlagens von SO₂ Sauerstoff auch in Form von atmosphärischer Luft eingesetzt werden. Diese wegen der Preisgünstigkeit bevorzugte Variante erzeugt sodann als Abgas Stickstoff mit nur ganz geringen Mengen an restlichem Sauerstoff. Diese Variante bringt als weiteren Vorteil den Verzicht von Sicherheitsmaßnahmen mit sich, die beim Umgang mit reinem Sauerstoff erforderlich sind. Selbstverständlich kann die atmosphärische Luft, beispielsweise bis zur Erreichung von Sicherheitsgrenzen, mit reinem Sauerstoff angereichert werden.

Die Reaktionstemperatur liegt in dem dem Fachmann bekannten Bereich von 30 bis 40°C.

Das erfindungsgemäße Verfahren kann in vorteilhafter Weise kontinuierlich durchgeführt werden.

Die Vorteile des erfindungsgemäßen Verfahrens liegen zunächst in der hohen Selektivität auch im Umsatzbereich über 5 Mol-%. Weiterhin ist eine verbesserte technische Durchführbarkeit dadurch gegeben, daß keine Rühr- oder Vermischungsenergie zum Aufbau und zur Aufrechterhaltung einer Öl-Wasser-Emulsion nötig ist. Dadurch ist es möglich, die Begasungsstärke von Schwefeldioxid und Sauerstoff soweit zu vermindern und bei Anwesenheit anderer Durchmischungsorgane, beispielsweise eines einfachen Rührers, so einzustellen, daß in der oben beschriebenen Weise kein entsorgungsbedürftiges, insbesondere kein Schwefeldioxid enthaltendes Abgas entsteht. In der homogenen, wasserklaren Reaktionslösung liegt das im Verlaufe des Reaktionsfortschritts immer weiter gemäß obiger Formel zugesetzte Wasser ideal verteilt und in wesentlich geringerer Menge als üblich vor. Die Belichtung mit dem UV-Strahlung enthaltendem Licht wird dadurch wesentlich begünstigt.

Zur Aufarbeitung wird der homogenen, wasserklaren und farblosen Reaktionslösung soviel Wasser zugesetzt, daß sich ein Zweiphasensystem einstellt. Die Phasentrennung ist spontan und wegen der stärkeren Unterdrückung von Nebenprodukten mulmfrei. Die wäßrige Phase aus dieser Phasentrennung wird entnommen und in einer dem Fachmann bekannten Weise aufgearbeitet. Die Paraffinphase aus dieser Phasentrennung kann ohne Zwischenreinigung direkt wieder eingesetzt werden.

### Beispiel 1

In einer 700 ml-Photolysenapparatur wurden 425 g n-Paraffin (C₁₃-C₁₈ Schnitt) bei 35°C vorgelegt. Man gab 17,7 g Wasser zu und leitete unter Bestrahlung mit einem 50 W-Hg-Niederdruckbrenner stündlich 1,26 g Sauerstoff und 5,84 g Schwefeldioxid unter Rühren abgasfrei ein. Nach ca. 1,5 Stunden bildete sich aus der Emulsion der flüssigen Reaktionskomponenten eine wasserklare, homogene Lösung. Zur Aufrechterhaltung dieser homogenen Lösung wurden stündlich 9,9 g Wasser zugepumpt. Nach 5 Stunden wurde die Sulfoxidation bei einem Umsatz von 10 Mol -% beendet. Zusatz von 28,6 g Wasser bewirkte eine spontane mulmfreie Phasentrennung in 290 g Paraffinphase und 234 g Wasserphase. Die farblose Wasserphase enthielt 21,63 % Paraffinsulfonsäure und 1,92 % Paraffindisulfonsäure, das entsprach 8,15 Gew-% bezüglich der Gesamtsulfonsäuremasse.

### Beispiel 2

Analog Beispiel 1 wurden stündlich 4,66 g Luft und 5,7 g Schwefeldioxid eingeleitet. Nach ca. 1,5 Stunden wurden stündlich 6,1 g Wasser zugepumpt. Nach 5,8 Stunden betrug der Paraffinumsatz 8,5 %. Der Luftsauerstoff reagierte zu 90 %. Nach Zusatz von 34,6 g Wasser erhielt man eine farblose Wasserphase, die 20,6 % Paraffinsulfonsäure und 0,7 % Paraffindisulfonsäure (3,3 % der Gesamtsulfonsäuremasse) enthielt.

### Beispiel 3 (Vergleichsbeispiel)

Analog Beispiel 1 wurden in 5,4 Stunden die Einsatzkomponenten stündlich mit 1,18 g Sauerstoff und 5,46 g Schwefeldioxid begast. Nach dem Erreichen der homogenen Phase wurde jedoch kein weiteres Wasser zugepumpt. Die Reaktionslösung wurde trübe und färbte sich zunehmend gelb. Nach dem Ende der Sulfoxidation bei einem Paraffinumsatz von 10,8 % bewirkte Wasserzusatz die Bildung einer gelartigen Masse, die sich nur langsam in zwei Phasen trennte. Die gelb gefärbte Wasserphase enthielt neben 23,57 % Paraffinsulfonsäure bereits 2,84 % Paraffindisulfonsäure, das entsprach 10,75 Gew.-% bezüglich der Gesamtsulfonsäuremasse.

## Patentansprüche

1. Verfahren zur Herstellung von aliphatischen Sulfonsäuren durch Umsetzung von n-Paraffinen mit Schwefeldioxid und Sauerstoff in Gegenwart von Wasser und unter Belichtung mit UV-Strahlung enthaltendem Licht (Sulfoxidation), dadurch gekennzeichnet, daß die Sulfoxidation nach Anlaufen der Reaktion und Erreichung des stationären Zustandes in homogener Phase durchgeführt wird.

2. Verfahren nach Anspruch 1, dadurch gekennzeichnet, daß nach Erreichung des stationären Zustandes die homogene Phase durch den Gehalt der Reaktionsmischung an jeweils zugesetztem Wasser, bezogen auf die zu Reaktionsbeginn vorhandene Gewichtsmenge an Paraffin und die Gesamtmenge an zugesetztem Wasser, in Abhängigkeit vom Paraffinumsatz aufrechterhalten wird, wobei der Paraffinumsatz durch den Sauerstoffverbrauch gemessen wird und wobei die jeweilig zugesetzte Wassermenge vom jeweilig erreichten Paraffinumsatz in Mol-%, bezogen auf die zu Reaktionsbeginn vorhandene Gewichtsmenge an Paraffin, durch die Formel
H₂O[Gew.%] = Paraffinumsatz [Mol-%]·0,93 + (1,67±0,5),
verknüpft ist.

3. Verfahren nach Anspruch 2, dadurch gekennzeichnet, daß die Reaktion in einem Umsatzbereich von 0,5 bis 12 Mol-% durchgeführt wird.

4. Verfahren nach Anspruch 3, dadurch gekennzeichnet, daß die Reaktion in einem Umsatzbereich von 2,5 bis 10 Mol-% durchgeführt wird.

5. Verfahren nach Anspruch 1, dadurch gekennzeichnet, daß zum Anlaufen der Reaktion und bis zur Erreichung des stationären Zustandes 1,17 bis 5 Gew.-% Wasser vorgelegt werden bzw. im Reaktionsgemisch vorliegen.

6. Verfahren nach Anspruch 5, dadurch gekennzeichnet, daß 1,17 bis 4 Gew.-% Wasser vorgelegt werden bzw. im Reaktionsgemisch vorliegen.

7. Verfahren nach Anspruch 1, dadurch gekennzeichnet, daß der Sauerstoff im Form atmosphärischer Luft eingesetzt wird.

## Claims

1. Process for the preparation of aliphatic sulphonic acids by reacting n-paraffins with sulphur dioxide and oxygen in the presence of water and under irradiation by UV-containing light (sulphoxidation), characterised in that the sulphoxidation is carried out in homogeneous phase after the reaction has commenced and the stationary state has been reached.

2. Process according to Claim 1, characterised in that after the stationary state has been reached, the homogeneous phase is maintained using the content of water which has been added in each case to the reaction mixture, relative to the weight of paraffin present at the start of the reaction and the total amount of water added, as a function of the paraffin conversion, the paraffin conversion being measured by the oxygen consumption, and the amount of water added in each case being linked to the paraffin conversion in mol% which has been achieved in each case, relative to the weight of paraffin present at the start of the reaction, by the formula
H₂O[% by weight] = paraffin conversion [mol%] x 0.93 + (1.67±0.5)

3. Process according to Claim 2, characterised in that the reaction is carried out within a conversion range of 0.5 to 12 mol%.

4. Process according to Claim 3, characterised in that the reaction is carried out within a conversion range of 2.5 to 10 mol%.

5. Process according to Claim 1, characterised in that at the start of the reaction and until the stationary state has been reached, 1.17 to 5% by weight of water are initially charged or are present in the reaction mixture.

6. Process according to Claim 5, characterised in that 1.17 to 4% by weight of water are initially charged or are present in the reaction mixture.

7. Process according to Claim 1, characterised in that the oxygen used is in the form of atmospheric air.

## Revendications

1. Procédé de préparation d'acides sulfoniques aliphatiques par réaction de n-paraffines avec le dioxyde de soufre et l'oxygène en présence d'eau et sous irradiation par de la lumière contenant des radiations UV (sulfoxydation), caractérisé en ce que, après le démarrage de la réaction et lorsqu'on a atteint l'état de régime, on procède à la sulfoxydation en phase homogène.

2. Procédé selon la revendication 1, caractérisé en ce que, lorsqu'on a atteint l'état de régime, on maintient l'état de phase homogène en agissant sur la teneur du mélange de réaction en eau ajoutée dans chaque cas, par rapport au poids de paraffine présent au début de la réaction et à la quantité totale d'eau ajoutée, en fonction du taux de conversion de la paraffine, ce dernier étant déterminé par la consommation d'oxygène, la quantité d'eau ajoutée dans chaque cas étant fonction du taux de conversion de la paraffine atteint dans chaque cas, en mol% par rapport au poids de paraffine présent au début de la réaction, conformément à l'équation:
H₂O, % en poids = taux de conversion de la paraffine, mol%, x 0,93 + (1,67 ± 0,5).

3. Procédé selon la revendication 2, caractérisé en ce que la réaction est effectuée dans l'intervalle des taux de conversion de 0,5 à 12 mol%.

4. Procédé selon la revendication 3, caractérisé en ce que la réaction est effectuée dans l'intervalle des taux de conversion de 2,5 à 10 mol%.

5. Procédé selon la revendication 1, caractérisé en ce que, au démarrage de la réaction et jusqu'à ce qu'on ait atteint l'état de régime, on opère après introduction de 1,17 à 5 % en poids d'eau ou on ajoute de 1,17 à 5 % en poids d'eau au mélange de réaction.

6. Procédé selon la revendication 5, caractérisé en ce que l'on opère après introduction de 1,17 à 4 % en poids d'eau ou bien on joute de 1,17 à 4 % en poids d'eau au mélange de réaction.

7. Procédé selon la revendication 1, caractérisé en ce que l'oxygène est utilisé sous la forme d'air atmosphérique.
